**Europäisches Patentamt**

**European Patent Office**

**Office européen des brevets**

(19)

(11) Veröffentlichungsnummer: **0 291 860 B1**

(12) # EUROPÄISCHE PATENTSCHRIFT

(45) Veröffentlichungstag der Patentschrift: **16.10.91**

(21) Anmeldenummer: **88107662.4**

(22) Anmeldetag: **13.05.88**

Die Akte enthält technische Angaben, die nach dem Eingang der Anmeldung eingereicht wurden und die nicht in dieser Patentschrift enthalten sind.

(51) Int. Cl.5: **C07C 19/08, C07C 17/22, C07F 3/06**

(54) **Verfahren zur Herstellung von CF3I.**

(30) Priorität: **22.05.87 DE 3717358**

(43) Veröffentlichungstag der Anmeldung:
**23.11.88 Patentblatt 88/47**

(45) Bekanntmachung des Hinweises auf die Patenterteilung:
**16.10.91 Patentblatt 91/42**

(84) Benannte Vertragsstaaten:
**AT BE CH DE ES FR GB GR IT LI LU NL SE**

(56) Entgegenhaltungen:
**EP-A- 0 266 281**

**J. CHEM. SOC., 1955, S. 2901-2910**

**Römpp's Chemie-Lexikon, 8. Auflage, 1985, S. 3903**

**TETRAHEDRON Lett., 29, 1988, S. 1029-1030**

(73) Patentinhaber: **Kali-Chemie Aktiengesellschaft**
**Postfach 220, Hans-Böckler-Allee 20**
**W-3000 Hannover 1(DE)**

(72) Erfinder: **Naumann, Dieter**
**Tunnelweg 9**
**W-4600 Dortmond 41(DE)**
Erfinder: **Tyrra, Wieland**
**Schultenstrasse 24**
**W-4690 Herne(DE)**
Erfinder: **Kock, Birgit**
**Sendener Stiege 51**
**W-4400 Münster(DE)**
Erfinder: **Rudolph, Werner**
**Oderstrasse 71**
**W-3000 Hannover 71(DE)**
Erfinder: **Wilkes, Bernd**
**Feldstrasse 16**
**W-3014 Laatzen 4(DE)**

(74) Vertreter: **Lauer, Dieter, Dr.**
**c/o Kali-Chemie AG Postfach 220 Hans-Böckler-Allee 20**
**W-3000 Hannover 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Trifluoriodmethan sowie Verbindungen der allg. Formel

$CF_3 \cdot Zn \cdot X \cdot nL$ .

Trifluoriodmethan wird beispielsweise als Trifluormethylierungsreagenz, zur Darstellung von Trifluornitrosomethan oder als Plasma-Ätz-Gas eingesetzt.

Es sind mehrere Methoden zur Darstellung von $CF_3I$ bekannt. So wird nach Banks et.al., J. Chem. Soc. **1948**, Seite 2188 bei der als heftig beschriebenen Reaktion von $Cl_4$ mit $IF_5$ als ein Reaktionsprodukt $CF_3I$ - neben $CF_3H$ - isoliert. Eine weitere Möglichkeit zur Darstellung von $CF_3I$ besteht in der thermischen Zersetzung von $CF_3COOAg$ in Anwesenheit von Iod (A.L. Henne, W.G. Finnegan, J.Am.Chem. Soc. **72**, 3806 (1950). Diese Reaktion gerät jedoch leicht außer Kontrolle. Bekannt ist ferner die Möglichkeit, Trifluoriodmethan durch die Spaltung von $CF_3HgI$ oder $(CF_3)_2Hg$ mit Iod darzustellen (H.J. Emeléus, R.N. Haszeldine, J.Chem. Soc. **1949**, 2953). Beide Quecksilberverbindungen zeichnen sich durch hohe Toxizität aus und werden zudem aus $CF_3I$ und Quecksilber hergestellt. Zwar läßt sich $(CF_3)_2Hg$ auch durch Decarboxylierung von $(CF_3COO)_2Hg$ darstellen, aber auch diese Verbindung ist hochtoxisch und ihre Verwendung aus Gründen des Umweltschutzes wenig wünschenswert.

Die europäische Patentanmeldung EP-A-0 266 281, die ein älteres Recht nach Artikel 54 (3) EPÜ ist, beschreibt ein Verfahren zur Herstellung von $CF_3I$. Bei jenem Verfahren wird ein Metall, insbesondere Zink, in einem aprotischen, polaren Lösungsmittel zunächst mit Schwefeldioxid oder Alkalidithionit versetzt. Anschließend wird dem Gemisch Trifluormethylbromid zugesetzt. Bei der anschließenden Zugabe von Jod bildet sich Triflourjodmethan. In der Patentanmeldung EP-A-0 266 281 wird hervorgehoben, daß die Reihenfolge der Zugabe von Schwefeldioxid bzw. Alkalidithionit und von Trifluormethylbromid von essentieller Bedeutung ist. Irgendwelche Hinweise auf ein Verfahren zur Herstellung von $CF_3I$ ohne jeden Zusatz von Schwefeldioxid oder Alkalidithionit finden sich nicht.

In jener europäischen Patentanmeldung finden sich auch keine Hinweise auf Konstitution oder gar Isolierbarkeit etwaiger Zwischenprodukte. Insbesondere findet sich kein Hinweis auf die Isolierung von Verbindungen der allgemeinen Formel $CF_3 \cdot Zn \cdot X \cdot nL$.

Bekannte Methoden zur Darstellung von $CF_3I$ haben den Nachteil, daß sie schwer zu beherrschende Reaktionsbedingungen erfordern, $CF_3I$ als Ausgangsprodukt voraussetzen und damit wirtschaftlich unvorteilhaft sind und/oder ökologische Probleme mit sich bringen.

Hier will die Erfindung Abhilfe schaffen, indem sie eine wirtschaftliche und gleichzeitig ökologisch vertretbare Methode zur Herstellung von $CF_3I$ vorstellt.

Die Aufgabe der Erfindung wird gelöst durch

a) Umsetzung von $CF_3X$ ohne Zusatz von $SO_2$ oder Alkalidithionit mit einem Metall Mt aus der Gruppe Magnesium, Zink, Cadmium, Wismut, Zinn in einem Lösungsmittel

b) Umsetzung des resultierenden Reaktionsgemisches mit einer Verbindung I-Y zu $CF_3I$, wobei X die Bedeutung Halogen, insbesondere Chlor oder Brom besitzt, und Y entweder Iod oder ein gegenüber Iod elektronegativer Ligand ist.

In bevorzugter Weise wird beim erfindungsgemäßen Verfahren in Stufe a) das Metall Zink verwendet.

Eine Abänderung des Verfahrens zur Herstellung von $CF_3I$ ist gekennzeichnet durch

a) Umsetzung von $CF_3X$ ohne Zusatz von $SO_2$ oder Alkalidithionit mit Zink in einem Lösungsmittel, wobei vor, während oder nach der Umsetzung ein komplexierendes Agens L dem Reaktionsgemisch zugesetzt wird

a1) Isolieren der gebildeten Organometallverbindung der allgemeinen Formel (I)

$CF_3 \cdot Zn \cdot X \cdot nL$    (I)

b) Umsetzung der in Stufe a1) isolierten Verbindung der Formel (I) mit einer Verbindung I-Y zu $CF_3I$, wobei X und Y die vorstehend benannte Bedeutung besitzen, n die Zahl 1 oder 2 ist und L das komplexierende Agens L bedeutet.

Bei den Verbindungen der allgemeinen Formel (I) besitzt X die Bedeutung Halogen, vorzugsweise Chlor oder Brom: n bedeutet die Zahl 1 oder 2 und L ein komplexierendes Agens L.

Das komplexierende Agens L kann eine ein- oder mehrzähnig komplexierende Verbindung und anorganischer oder organischer Natur sein. Vorzugsweise ist L eine organische Verbindung. Insbesondere bevorzugt ist L ein organisches, polares, aprotisches komplexierendes Agens. Gut geeignet als komplexie-

EP 0 291 860 B1

rendes Agens L sind Verbindungen aus der Gruppe der cyclischen oder acyclischen Ether, ggf. substituierte Carbonsäureamide, ggf. substituierte Lactame, Nitrile, Sulfoxide, Amine und/oder Pyridine. Als in Frage kommende komplexierende Agentien sind beispielhaft zu nennen Tetrahydrofuran, Diethylether, N,N-Dimethylformamid, N,N-Dimethylacetamid, N-Methylpyrrolidon, N-Ethylpyrrolidon, Acetonitril, Benzonitril, Triethylamin, Dimethylsulfoxid, Pyridin, substituiertes Pyridin wie z.B. Methylpyridin. Mehrzähnig komplexierende Agentien sind weniger bevorzugt, können aber auch eingesetzt werden; aus der Vielzahl der dem Fachmann bekannten mehrzähnigen Liganden seien Dioxan, Ethylenglycoldimethyläther, ggf. substituierte Diamine sowie Dipyridil erwähnt.

Die isolierten Zinkverbindungen der allgemeinen Formel (I), wobei X die Bedeutung Halogen, vorzugsweise Chlor oder Brom: n die Zahl 2 bedeutet und L ein komplexierendes Agens L aus der Gruppe der ggf. substituierten Carbonsäureamide ist, sind neu und ebenfalls Gegenstand der Erfindung.

Vorzugsweise ist L ein N-disubstituiertes Carbonsäureamid, insbesondere N,N-Dimethylformamid (DMF).

In den Verbindungen der allgemeinen Formel (I) besitzt n vorzugsweise die Bedeutung 2.

Ganz besonders bevorzugt ist die Verbindung

$$CF_3 \cdot Zn \cdot Br \cdot 2\ DMF\ .$$

Die Verbindungen der allgemeinen Formel (I) können hergestellt werden durch Umsetzung von $CF_3X$ mit Zink in einem Lösungsmittel, wobei vor, während oder nach der Umsetzung ein komplexierendes Agens L dem Reaktionsgemisch zugesetzt wird und man die gebildete Organometallverbindung mit der allgemeinen Formel (I) isoliert. Vorzugsweise arbeitet man unter Ausschluß von Feuchtigkeit und Sauerstoff. Dies kann durch den Einsatz inerter Gase (Schutzgase), z.B. Stickstoff, bewirkt werden. Die Isolierung der Verbindungen der Formel (I) erfolgt nach Methoden, die dem Fachmann an sich bekannt sind, beispielsweise durch Entfernen des Lösungsmittels oder durch Kristallisation. Sofern man bei erhöhtem Druck gearbeitet hat, ist es zunächst vorteilhaft, eine Druckentspannung auf den Normaldruck vorzunehmen. Ferner kann es vorteihaft sein, die Reaktionsmischung von nicht umgesetztem Zink zu befreien - z.B. durch Filtration oder Dekantieren. Die Temperatur der Reaktionsmischung sollte bei der Isolierung der Verbindungen eine Temperatur von etwa 60 °C, vorzugsweise etwa 50 °C, nicht übersteigen. Das Entfernen von Lösungsmitteln kann durch Anlegen eines verminderten Druckes unterstützt werden. Die Verbindungen der allgemeinen Formel (I) bleiben als kristalline Feststoffe zurück; sie können von etwaig anhaftenden Lösungsmittelresten durch Waschen, beispielsweise mit $CCl_3F$ oder Toluol, befreit werden. Zur Vermeidung von Zersetzungsreaktionen werden die Verbindungen unter Schutzgasatmosphäre, z.B. Stickstoff, aufbewahrt und weiterverarbeitet.

Verwendbar sind die Verbindungen der allg. Formel (I) beispielsweise als Zwischenprodukte bei der Herstellung von $CF_3I$.

Die Reaktion gemäß Stufe a) und/oder Stufe b) wird in einem Lösungsmittel durchgeführt. Als bevorzugte Lösungsmittel kommen polare aprotische Lösungsmittel, ggf. Lösungsmittelgemische in Betracht. Sie werden vorzugsweise aus der Gruppe der cyclischen oder acyclischen Ether, ggf. substituierten Carbonsäureamide, ggf. substituierte Lactame, Nitrile, Sulfoxide, Amine und/oder Pyridine ausgewählt. Beispielhaft zu nennen sind Tetrahydrofuran, Dioxan, Diethylether, N,N-Dimethylformamid, N,N-Dimethylacetamid, N-Methylpyrrolidon, N-Ethylpyrrolidon, Acetonitril, Benzonitril, Triethylamin, Dimethylsulfoxid, Pyridin und/oder substituiertes Pyridin wie z.B. Methylpyridin.

Bevorzugte Lösungsmittel sind N,N-disubstituierte Carbonsäureamide, insbesondere N,N-Dimethylformamid (DMF).

Sofern man die Herstellung von $CF_3I$ nach dem abgeänderten Verfahren durchführt, welches die Isolierung der Verbindungen der allgemeinen Formel (I) beinhaltet, besteht eine bevorzugte Variante darin, daß Lösungsmittel und komplexierendes Agens L identisch sind. Als Lösungsmittel und als komplexierendes Agens L werden bevorzugt N,N-disubstituierte Carbonsäureamide, insbesondere bevorzugt N,N-Dimethylformamid, eingesetzt.

Die Umsetzung gemäß Stufe a) kann in an sich bekannter Weise durch Einwirkung von Ultraschall z.B. gemäß EP-A-0 082 252 oder EP-A-0 206 950 unterstützt werden.

Im allgemeinen wird das vorzugsweise pulverförmige Metall im Lösungsmittel suspendiert und die, ggf. in einem Lösungsmittel gelöste, die $CF_3$-Gruppe enthaltende Komponente $CF_3X$ zwischen -60 °C und +75 °C, insbesondere zwischen Raumtemperatur und +70 °C, zur Metallsuspension zugegeben.

In einer Variante wird die Umsetzung gemäß Stufe a) in Gegenwart katalytischer Mengen an einem, in metallischer und/oder ionischer Form eingesetzten Metall durchgeführt, das elektropositiver als das für die Umsetzung in Stufe a) eingesetzte Metall Mt ist. In bevorzugter Weise verwendet man eines oder mehrere

3

Metalle aus der Gruppe Eisen, Blei, Kupfer. Vorteilhaft werden Salze verwendet, die in einer katalytisch wirksamen Menge im verwendeten Lösungsmittel löslich sind. Als Gegenion des Metallkations dienen Halogenide, vorzugsweise Iodid, oder organische Anionen, wie Acetat.

In einer weiteren Variante führt man die Umsetzung gemäß Stufe a) in Anwesenheit von Verbindungen durch, die als Katalysatoren von Grignardreaktionen bekannt sind, wie Iod oder Tetrachlorkohlenstoff.

In einer weiteren Variante wird die Umsetzung nach Stufe a) elektrochemisch durchgeführt, wie sie z.B. bei J.J. Habeeb et al., J. Organometal. Chem. **185** (1980), Seite 117 ff beschrieben ist. Diese Variante kann insbesondere auch in Kombination mit dem oben erwähnten Zusatz von Katalysatoren für Grignard-Reaktionen durchgeführt werden.

Die Umsetzung gemäß Stufe a) kann bei Normaldruck oder höherem Druck bis maximal 50 bar (absolut) durchgeführt werden. Bevorzugt arbeitet man bei Drucken, die zwischen 1 bar (absolut) und dem durch die Reaktionstemperatur bestimmten Gleichgewichtsdruck liegen.

In allen Varianten ist es vorteilhaft, das eingesetzte Metall Mt vollständig zur Reaktion zu bringen, bevor die Umsetzung nach Stufe b) durchgeführt wird.

Das gemäß Stufe a) hergestellte Reaktionsgemisch wird gemäß Stufe b) mit einer ggf. in einem Lösungsmittel gelösten Verbindung der Formel I-Y umgesetzt.

Sofern man nach der Abänderung des Verfahrens arbeitet, setzt man die in Stufe a1) isolierten Verbindungen der allgemeinen Formel (I), vorzugsweise nach ihrer Wiederauflösung in einem Lösungsmittel, gemäß Stufe b) mit einer ggf. in einem Lösungsmittel gelösten Verbindung der Formel I-Y um.

Die Reaktionstemperatur kann im Bereich zwischen -30 $^\circ$C und +50 $^\circ$C, bevorzugt bei Raumtemperatur liegen. In der Formel I-Y bedeutet Y entweder Iod oder einen gegenüber Iod elektronegativen Liganden. Bevorzugt hat Y die Bedeutung Halogen, insbesondere Chlor, Brom, oder Iod.

Das im Verlauf der Umsetzung entstehende Trifluoriodmethan kann durch Destillation, insbesondere Vakuumdestillation, isoliert werden.

Das erfindungsgemäße Verfahren zur Herstellung von Trifluoriodmethan besitzt überraschende Vorteile.
- die Umsetzung gemäß Stufe a) und b) ist gut kontrollierbar und damit gut in den technischen Maßstab umsetzbar
- die Herstellung erfolgt in ökologisch vertretbarer Weise
- die Ausbeute an $CF_3I$ ist hoch (90 % und mehr, bezogen auf eingesetztes ICl als Verbindung der Formel I-Y)
- die Selektivität ist hoch
- das Produkt ist frei von Verunreinigungen.

Das abgeänderte Verfahren zur Herstellung von $CF_3I$, in welchem man zunächst die Verbindungen der allgemeinen Formel (I) isoliert und vorzugsweise nach ihrer Wiederauflösung in einem Lösungsmittel, weiter umsetzt, bietet zusätzlich den Vorteil, daß die Reaktionspartner in der Stufe b) in exakt dosierbarer Menge eingesetzt werden können. Die Reaktionskontrolle durch Bromid-Bestimmung erübrigt sich bei dieser Variante.

Zur weiteren Erläuterung der Erfindung sollen die folgenden Beispiele dienen, ohne sie in ihrem Umfang zu beschränken.

Beispiel 1 (Durchführung Stufe a):

10 g Zink werden in 200 g DMF suspendiert und mit 1 g Iod versetzt. Bei Raumtemperatur werden anschließend 10 g $CF_3Br$ unter leichtem Überdruck (1,5 bar absolut) eingeleitet. In die Lösung werden eine Platin-Kathode und Zink-Anode eingetaucht und eine Spannung von 15 V bei einer Stromstärke von 250 mA angelegt. Mit fallendem Druck wird neues $CF_3Br$ eingeleitet, bis das gesamte suspendierte Zink verbraucht ist. Da die Zink-Anode ebenfalls verbraucht wird, ist ein $CF_3Br$-Überschuß zu verwenden. Die Reaktionskontrolle erfolgt durch Bromid-Bestimmungen (theoretischer Bromid-Gehalt: 5,2 %; gefunden 4,9-5,0 %).

Beispiel 2 (Durchführung Stufe b):

Im Reaktor werden 200 g einer Grignard-DMF-Lösung, die 15,1 Gew.-% $Br^-$ = 13,0 Gew.-% $CF_3^-$ enthält, bei Raumtemperatur vorgelegt. Anschließend wird Iodmonochlorid (60 g, gelöst in 30 ml DMF) langsam zugegeben. Das entstehende $CF_3I$ wird während der exothermen Reaktion abdestilliert und in nachgeschalteten Kühlfallen kondensiert.
Ausbeute an $CF_3I$: 92 % (bezogen auf eingesetztes ICl).

Beispiel 3:

In 100 ml DMF werden 10 g Zn-Pulver suspendiert. Nach Zugabe katalytischer Mengen an Iod wird bei -30 °C ein Überschuß an $CF_3Br$ gelöst. Die Umsetzung wird bei Raumtemperatur im Ultraschallbad durchgeführt. Zum Reaktionsgemisch werden anschließend äquimolare Mengen ICl zugegeben und das gebildete $CF_3I$ destillativ isoliert.

Beispiel 4:

In 100 ml trockendem Dimethylformamid (DMF) werden 10 g Zn-Pulver suspendiert und auf ca. 30 °C temperiert. In die flüssige Phase wird anschließend unter sehr kräftigem Rühren $CF_3Br$ bis zu einem maximalen Überdruck von ca. 1 bar eingeleitet (geschlossenes System). Hierbei wird die $CF_3Br$-Dosierung so eingestellt, daß die Reaktionstemperatur 40 °C nicht übersteigt. Nach beendeter Reaktion wird entspannt und die Reaktionsmischung durch Filtration von nicht umgesetztem Zink befreit. Unter vermindertem Druck wird das Lösemittel abgezogen, wobei die Destillationstemperatur unterhalb etwa 50 bis 60 °C gehalten wird.

Die hergestellte Zinkverbindung der Formel $CF_3 \cdot Zn \cdot Br \cdot 2$ DMF bleibt als kristalliner Feststoff zurück und kann ggf. durch Waschen mit $CCl_3F$ oder Toluol gereinigt werden.

Analysendaten:

| | |
|---|---|
| Fp : | 99 - 100 °C |
| NMR-Daten: | $d\,(^{19}F) = -42{,}6$ ppm $\qquad (CF_3)$ |
| | $d\,(^{13}C) = 145{,}5$ ppm, Quartett $(CF_3)$ |
| | $^{1}J\,(^{19}F - {}^{13}C) = 358{,}3$ Hz |
| | $d\,(^{13}C) = 165{,}8$ ppm $\qquad (C=0)$ |
| | $d\,(^{13}C) = 37{,}6$ ppm $\qquad (CH_3)$ |
| | $d\,(^{13}C) = 32{,}3$ ppm $\qquad (CH_3)$ |
| Elementaranalyse: | $C_7 H_{14} Br F_3 N_2 O_2 Zn$ (360,49) |

| | C | H | N |
|---|---|---|---|
| gefunden (%) | 20,3 | 3,6 | 7,5 |
| berechnet (%) | 23,3 | 3,9 | 7,8 |

Beispiel 5:

Unter Schutzgasatmosphäre werden 30 g der kristallinen Organozinkverbindung $CF_3 \cdot Zn \cdot Br \cdot 2$ DMF in 100 ml getrocknetem Dimethylformamid gelost. Zu der Lösung gibt man äquimolare Mengen ICl; das im Verlauf der Reaktion gebildete $CF_3I$ wird destillativ isoliert.

Das gemäß den Beispielen hergestellte $CF_3I$ ist gemäß infrarotspektroskopischer und gaschromatographischer Überprüfung frei von Verunreinigungen wie z.B. $CF_3H$.

**Patentansprüche**
**Patentansprüche für folgende Vertragsstaaten : AT, BE, CH, DE, FR, GB, IT, LI, LU, NL, SE, GR**

1. Verfahren zur Herstellung von $CF_3I$, gekennzeichnet durch
   a) Umsetzung von $CF_3X$ ohne Zusatz von $SO_2$ oder Alkalidithionit mit einem Metall Mt aus der Gruppe Magnesium, Zink, Cadmium, Wismut, Zinn in einem Lösungsmittel
   b) Umsetzung des resultierenden Reaktionsgemisches mit einer Verbindung I-Y zu $CF_3I$,

   wobei X die Bedeutung Halogen, insbesondere Chlor oder Brom besitzt und Y entweder Iod oder ein gegenüber Iod elektronegativer Ligand ist.

2. Verfahren gemäß Anspruch 1, dadurch gekennzeichnet, daß als Metall Zink eingesetzt wird,

3. Abänderung des Verfahrens gemäß Anspruch 2, gekennzeichnet durch

   a) Umsetzung von $CF_3X$ mit Zink ohne Zusatz von $SO_2$ oder Alkalidithionit in einem Lösungsmittel, wobei vor, während oder nach der Umsetzung ein komplexierendes Agens L dem Reaktionsgemisch zugesetzt wird

a1) Isolieren der gebildeten Organometallverbindung der allg. Formel (I)

$$CF_3 \cdot Zn \cdot X \cdot nL \quad (I)$$

b) Umsetzung der in Stufe a1) isolierten Verbindung der Formel (I) mit einer Verbindung I-Y zu $CF_3I$, wobei X und Y die vorstehend benannte Bedeutung besitzen, n die Zahl 1 oder 2 ist und L das komplexierende Agens L bedeutet.

4. Verfahren gemäß einem der vorhergehenden Ansprüche dadurch gekennzeichnet, daß die Umsetzung gemäß Stufe a) und/oder Stufe b) in einem polaren, aprotischen Lösungsmittel oder einem Gemisch aus solchen durchgeführt wird.

5. Verfahren gemäß Anspruch 4, dadurch gekennzeichnet, daß ein oder mehrere Lösungsmittel aus der Gruppe der cyclischen oder acyclischen Ether, der ggf. substituierten Carbonsäureamide, der ggf. substituierten Lactame, Nitrile, Sulfoxide, Amine und/oder Pyridine eingesetzt werden.

6. Verfahren gemäß Anspruch 3 bis 6, dadurch gekennzeichnet, daß das komplexierende Agens L ein Ligand aus der Gruppe der cyclischen oder acyclischen Ether, der ggf. substituierten Carbonsäureamide, der ggf. substituierten Lactame, Nitrile, Sulfoxide, Amine und/oder Pyridine ist.

7. Verfahren gemäß Anspruch 3 bis 6, dadurch gekennzeichnet, daß Losungsmittel und komplexierendes Agens L identisch sind.

8. Verfahren gemäß Anspruch 7, dadurch gekennzeichnet, daß N-disubstituierte Carbonsäureamide, insbesondere N,N-Dimethylformamid (DMF) als Lösungsmittel und komplexierendes Agens L eingesetzt werden.

9. Verfahren gemäß einem der vorhergehenden Ansprüche, dadurch gekennzeichnet, daß man die Stufe a) in Gegenwart katalytischer Mengen an einem Metall durchführt, das elektropositiver als das für die Umsetzung eingesetzte Metall ist und das in metallischer und/oder ionischer Form eingesetzt wird.

10. Verfahren gemäß einem der vorhergehenden Ansprüche, dadurch gekennzeichnet, daß man die Stufe a) in Gegenwart von Grignardreaktionen aktivierenden Zusätzen, insbesondere halogenhaltigen Mitteln, wie Iod, $CCl_4$ durchführt.

11. Verfahren gemäß einem der vorhergehenden Ansprüche, dadurch gekennzeichnet, daß man die Stufe a) auf elektrochemischem Wege durchführt.

12. Verfahren gemäß einem der vorhergehenden Ansprüche, dadurch gekennzeichnet, daß der Ligand Y die Bedeutung Halogen, insbesondere Chlor; Brom, Iod besitzt.

13. Verfahren gemäß einem der vorhergehenden Ansprüche, dadurch gekennzeichnet, daß die Stufe a) bei erhöhtem Druck im Autoklaven bis max. 50 bar absolut, vorzugsweise bei Drucken von 1 bar absolut bis zu dem durch die Reaktions-Temperatur bestimmten Gleichgewichtsdruck durchgeführt wird.

14. Verbindungen der allg. Formel (I)

$$CF_3 \cdot Zn \cdot X \cdot nL \quad (I)$$

wobei X Halogen, vorzugsweise Chlor oder Brom bedeutet, n die Zahl 2 und

L ein komplexierendes Agens aus der Gruppe der ggf. substituierten Carbonsäureamide ist.

15. Verbindungen gemäß Anspruch 14, dadurch gekennzeichnet, daß L ein N-disubstituiertes Carbonsäureamid, insbesondere N,N-Dimethylformamid (DMF) ist.

EP 0 291 860 B1

**16.** Verbindung gemäß Anspruch 15 der Formel

CF₃ • Zn • Br • 2 DMF

**Patentansprüche für folgenden Vertragsstaat : ES**

**1.** Verfahren zur Herstellung von CF₃I, gekennzeichnet durch
a) Umsetzung von CF₃X ohne Zusatz von SO₂ oder Alkalidithionit mit einem Metall Mt aus der Gruppe Magnesium, Zink, Cadmium, Wismut, Zinn in einem Lösungsmittel
b) Umsetzung des resultierenden Reaktionsgemisches mit einer Verbindung I-Y zu CF₃ I,

wobei X die Bedeutung Halogen, insbesondere Chlor oder Brom besitzt und Y entweder Iod oder ein gegenüber Iod elektronegativer Ligand ist.

**2.** Verfahren gemäß Anspruch 1, dadurch gekennzeichnet, daß als Metall Zink eingesetzt wird.

**3.** Abänderung des Verfahrens gemäß Anspruch 2, gekennzeichnet durch

a) Umsetzung von CF₃X mit Zink ohne Zusatz von SO₂ oder Alkalidithionit in einem Lösungsmittel, wobei vor, während oder nach der Umsetzung ein komplexierendes Agens L dem Reaktionsgemisch zugesetzt wird .
a1) Isolieren der gebildeten Organometallverbindung der allg. Formel (I)

CF₃ • Zn • X • nL      (I)

b) Umsetzung der in Stufe a1) isolierten Verbindung der Formel (I) mit einer Verbindung I-Y zu CF₃I, wobei X und Y die vorstehend benannte Bedeutung besitzen, n
die Zahl 1 oder 2 ist und L das komplexierende Agens L bedeutet.

**4.** Verfahren gemäß einem der vorhergehenden Ansprüche dadurch gekennzeichnet, daß die Umsetzung gemäß Stufe a) und/oder Stufe b) in einem polaren, aprotischen Lösungsmittel oder einem Gemisch aus solchen durchgeführt wird.

**5.** Verfahren gemäß Anspruch 4, dadurch gekennzeichnet, daß ein oder mehrere Lösungsmittel aus der Gruppe der cyclischen oder acyclischen Ether, der ggf. substituierten Carbonsäureamide, der ggf. substituierten Lactame, Nitrile, Sulfoxide, Amine und/oder Pyridine eingesetzt werden.

**6.** Verfahren gemäß Anspruch 3 bis 6, dadurch gekennzeichnet, daß das komplexierende Agens L ein Ligand aus der Gruppe der cyclischen oder acyclischen Ether, der ggf. substituierten Carbonsäureamide, der ggf. substituierten Lactame, Nitrile, Sulfoxide, Amine und/oder Pyridine ist.

**7.** Verfahren gemäß Anspruch 3 bis 6, dadurch gekennzeichnet, daß Lösungsmittel und komplexierendes Agens L identisch sind.

**8.** Verfahren gemäß Anspruch 7, dadurch gekennzeichnet, daß N-disubstituierte Carbonsäureamide, insbesondere N,N-Dimethylformamid (DMF) als Lösungsmittel und komplexierendes Agens L eingesetzt werden.

**9.** Verfahren gemäß einem der vorhergehenden Ansprüche, dadurch gekennzeichnet, daß man die Stufe a) in Gegenwart katalytischer Mengen an einem Metall durchführt, das elektropositiver als das für die Umsetzung eingesetzte Metall ist und das in metallischer und/oder ionischer Form eingesetzt wird.

**10.** Verfahren gemäß einem der vorhergehenden Ansprüche, dadurch gekennzeichnet, daß man die Stufe a) in Gegenwart von Grignardreaktionen aktivierenden Zusätzen, insbesondere halogenhaltigen Mitteln, wie Iod, CCl₄ durchführt.

**11.** Verfahren gemäß einem der vorhergehenden Ansprüche, dadurch gekennzeichnet, daß man die Stufe

7

a) auf elektrochemischem Wege durchführt.

12. Verfahren gemäß einem .der vorhergehenden Ansprüche, dadurch gekennzeichnet, daß der Ligand Y die Bedeutung Halogen, insbesondere Chlor; Brom, Iod besitzt.

13. Verfahren gemäß einem der vorhergehenden Ansprüche, dadurch gekennzeichnet, daß die Stufe a) bei erhöhtem Druck im Autoklaven bis max. 50 bar absolut, vorzugsweise bei Drucken von 1 bar absolut bis zu dem durch die Reaktions-Temperatur bestimmten Gleichgewichtsdruck durchgeführt wird.

14. Verfahren zur Herstellung von Verbindungen der allg. Formel (I)

$$CF_3 \cdot Zn \cdot X \cdot nL \quad (I)$$

wobei X Halogen, vorzugsweise Chlor oder Brom bedeutet, n die Zahl 2 und

L ein komplexierendes Agens aus der Gruppe der ggf. substituierten Carbonsäureamide ist, gekennzeichnet durch

a) Umsetzung von $CF_3X$ mit Zink ohne Zusatz von $SO_2$ oder Alkalidithionit in einem Lösungsmittel, wobei vor, während oder nach der Umsetzung ein komplexierendes Agens L dem Reaktionsgemisch zugesetzt wird
a1) Isolieren der gebildeten Organometallverbindung der allg. Formel (I).

15. Verfahren gemäß Anspruch 14, dadurch gekennzeichnet, daß L ein N-disubstituiertes Carbonsäureamid, insbesondere N,N-Dimethylformamid (DMF) ist.

16. Verfahren gemäß Anspruch 15, dadurch gekennzeichnet, daß X Brom bedeutet, L Dimethylformamid (DMF) ist und die hergestellte Verbindung die Formel

$$CF_3 \cdot Zn \cdot Br \cdot 2 \, DMF$$

besitzt.

## Claims
**Claims for the following Contracting States : AT, BE, CH, DE, FR, GB, IT, LI, LU, NL, SE, GR**

1. Method for the preparation of $CF_3I$, characterised by

a) reaction of $CF_3X$, without the addition of $SO_2$ or alkali dithionite, with a metal Mt from the group magnesium, zinc, cadmium, bismuth, tin, in a solvent
b) reaction of the resulting reaction mixture with a compound I-Y to form $CF_3I$,

wherein X has the meaning halogen, in particular chlorine or bromine, and Y is either iodine or a ligand which is electronegative with respect to iodine.

2. Method according to Claim 1, characterised in that zinc is used as the metal.

3. Modification of the method according to Claim 2, characterised by

a) reaction of $CF_3X$ with zinc, without the addition of $SO_2$ or alkali dithionite, in a solvent, whereby a complexing agent L is added to the reaction mixture before, during or after the reaction
a1) isolation of the resulting organometallic compound of the general Formula (I)

$$CF_3 \cdot Zn \cdot X \cdot nL \quad (I)$$

b) reaction of the compound of Formula (I) which was isolated in stage a1) with a compound I-Y to form $CF_3I$, wherein X and Y have the meanings given above, n is the number 1 or 2 and L is the complexing agent L.

4. Method according to one of the preceding Claims, characterised in that the reaction of stage a) and/or stage b) is carried out in a polar, aprotic solvent or a mixture of such solvents.

5. Method according to Claim 4, characterised in that one or more solvents from the group of cyclic or acyclic ethers, the optionally substituted carboxylic acid amides, the optionally substituted lactams, nitriles, sulphoxides, amines and/or pyridines, are used.

6. Method according to Claims 3 to 6, characterised in that the complexing agent L is a ligand from the group of cyclic or acyclic ethers, the optionally substituted carboxylic acid amides, the optionally substituted lactams, nitriles, sulphoxides, amines and/or pyridines.

7. Method according to Claims 3 to 6, characterised in that the solvent and complexing agent L are identical.

8. Method according to Claim 7, characterised in that N-disubstituted carboxylic acid amides, in particular N,N-dimethyl formamide (DMF), are used as the solvent and complexing agent L.

9. Method according to one of the preceding Claims, characterized in that stage a) is performed in the presence of catalytic quantities of a metal which is more electropositive than the metal used for the reaction and which is used in metallic and/or ionic form.

10. Method according to one of the preceding Claims, characterised in that stage a) is performed in the presence of additives which activate Grignard reactions, in particular halogen-containing agents, such as iodine, $CCl_4$.

11. Method according to one of the preceding Claims, characterized in that stage a) is performed electrochemically.

12. Method according to one of the preceding Claims, characterized in that the ligand Y has the meaning halogen, in particular chlorine, bromine, iodine.

13. Method according to one of the preceding Claims, characterized in that stage a) is performed at increased pressure in an autoclave up to a maximum of 50 bar absolute, preferably at pressures of 1 bar absolute up to the equilibrium pressure determined by the reaction temperature.

14. Compounds of the general Formula (I)

$$CF_3 \cdot Zn \cdot X \cdot nL \qquad (I)$$

wherein X is halogen, preferably chlorine or bromine, n is the number 2 and

L is a complexing agent from the group of optionally substituted carboxylic acid amides.

15. Compounds according to Claim 14, characterised in that L is an N-disubstituted carboxylic acid amide, in particular N,N-dimethyl formamide (DMF).

16. Compound according to Claim 15 of the formula

$$CF_3 \cdot Zn \cdot Br \cdot 2\,DMF .$$

**Claims for the following Contracting State : ES**

1. Method for the preparation of $CF_3I$, characterised by

a) reaction of $CF_3X$, without the addition of $SO_2$ or alkali dithionite, with a metal Mt from the group magnesium, zinc, cadmium, bismuth, tin, in a solvent

EP 0 291 860 B1

b) reaction of the resulting reaction mixture with a compound I-Y to form $CF_3I$,

wherein X has the meaning halogen, in particular chlorine or bromine, and Y is either iodine or a ligand which is electronegative with respect to iodine.

2. Method according to Claim 1, characterised in that zinc is used as the metal.

3. Modification of the method according to Claim 2, characterised by

   a) reaction of $CF_3X$ with zinc, without the addition of $SO_2$ or alkali dithionite, in a solvent, whereby a complexing agent L is added to the reaction mixture before, during or after the reaction
   a1) isolation of the resulting organometallic compound of the general Formula (I)

   $CF_3 \cdot Zn \cdot X \cdot nL$    (I)

   b) reaction of the compound of Formula (I) which was isolated in stage a1) with a compound I-Y to form $CF_3I$, wherein X and Y have the meanings given above, n is the number 1 or 2 and L is the complexing agent L.

4. Method according to one of the preceding Claims, characterised in that the reaction of stage a) and/or stage b) is carried out in a polar, aprotic solvent or a mixture of such solvents.

5. Method according to Claim 4, characterised in that one or more solvents from the group of cyclic or acyclic ethers, the optionally substituted carboxylic acid amides, the optionally substituted lactams, nitriles, sulphoxides, amines and/or pyridines, are used.

6. Method according to Claims 3 to 6, characterised in that the complexing agent L is a ligand from the group of cyclic or acyclic ethers, the optionally substituted carboxylic acid amides, the optionally substituted lactams, nitriles, sulphoxides, amines and/or pyridines.

7. Method according to Claims 3 to 6, characterised in that the solvent and complexing agent L are identical.

8. Method according to Claim 7, characterised in that N-disubstituted carboxylic acid amides, in particular N,N-dimethyl formamide (DMF), are used as the solvent and complexing agent L.

9. Method according to one of the preceding Claims, characterised in that stage a) is performed in the presence of catalytic quantities of a metal which is more electropositive than the metal used for the reaction and which is used in metallic and/or ionic form.

10. Method according to one of the preceding Claims, characterised in that stage a) is performed in the presence of additives which activate Grignard reactions, in particular halogen-containing agents, such as iodine, $CCl_4$.

11. Method according to one of the preceding Claims, characterised in that stage a) is performed electrochemically.

12. Method according to one of the preceding Claims, characterised in that the ligand Y has the meaning halogen, in particular chlorine, bromine, iodine.

13. Method according to one of the preceding Claims, characterised in that stage a) is performed at increased pressure in an autoclave up to a maximum of 50 bar absolute, preferably at pressures of 1 bar absolute up to the equilibrium pressure determined by the reaction temperature.

14. Method for producing compounds of the general Formula (I)

    $CF_3 \cdot Zn \cdot X \cdot nL$    (I)

10

wherein X is halogen, preferably chlorine or bromine, n is the number 2 and

L is a complexing agent from the group of optionally substituted carboxylic acid amides, characterised by

a) reaction of $CF_3X$ with zinc, without the addition of $SO_2$ or alkali dithionite, in a solvent, whereby a complexing agent L is added to the reaction mixture before, during or after the reaction
a1) isolation of the resulting organometallic compound of the general Formula (I).

15. Method according to Claim 14, characterised in that L is an N-disubstituted carboxylic acid amide, in particular N,N-dimethyl formamide (DMF).

16. Method according to Claim 15, characterised in that X is bromine, L is dimethyl formamide (DMF) and the compound produced has the formula

$CF_3 \cdot Zn \cdot Br \cdot 2\ DMF$ .

**Revendications**

**Revendications pour les Etats contractants suivants : AT, BE, CH, DE, FR, GB, IT, LI, LU, NL, SE, GR**

1. Procédé pour préparer $CF_3I$, caractérisé par :
   a) la réaction de $CF_3X$, sans addition de $SO_2$ ou de dithionite alcalin, avec un métal MT choisi dans le groupe constitué par le magnésium, le zinc, le cadmium, le bismuth, l'étain, dans un solvant,
   b) la réaction du mélange réactionnel résultant avec un composé I-Y pour obtenir $CF_3I$,
   X représentant un atome d'halogène, en particulier le chlore ou le brome, et Y représentant l'iode ou un ligand plus électro-négatif que l'iode.

2. Procédé selon la revendication 1, caractérisé en ce qu'on utilise le zinc comme métal.

3. Modification du procédé selon la revendication 2, caractérisée par :
   a) la réaction de $CF_3X$ avec le zinc, sans addition de $SO_2$ ni d'un dithionite alcalin, dans un solvant et l'addition avant, pendant ou après la réaction d'un agent L de complexation au mélange réactionnel,
   a1) l'isolement du composé organo-métallique de formule générale (I) ainsi formé :

   $CF_3 \cdot Zn \cdot X \cdot nL$     (I)

   b) la réaction du composé de formule (I), isolé à l'étape a1), avec un composé I-Y pour obtenir $CF_3I$, X et Y ayant le sens précité, n étant un nombre valant 1 ou 2 et L représentant l'agent L de complexation.

4. Procédé selon l'une des revendications précédentes, caractérisé en ce qu'on effectue la réaction selon l'étape a) et/ou l'étape b) dans un solvant aprotique polaire ou dans un mélange de tels solvants.

5. Procédé selon la revendication 4, caractérisé en ce qu'on utilise un ou plusieurs solvants choisis dans l'ensemble constitué par les éthers cycliques ou acycliques, les amides d'acides carboxyliques éventuellement substitués, les lactames, nitriles, sulfoxydes, amines et/ou pyridines éventuellement substitué(e)s.

6. Procédé selon l'une des revendications 3 à 6, caractérisé en ce que l'agent L de complexation est un ligand choisi dans l'ensemble formé par les éthers cycliques ou acycliques, les amides d'acides carboxyliques éventuellement substitués, les lactames, nitriles, sulfoxydes, amines et/ou pyridines éventuellement substitués.

7. Procédé selon l'une des revendications 3 à 6, caractérisé en ce que le solvant et l'agent L de complexation sont identiques.

**8.** Procédé selon la revendication 7, caractérisé en ce qu'on utilise des amides d'acides carboxyliques disubstitués sur l'azote, en particulier le N,N-diméthylformamide (DMF) comme solvant et comme agent L de complexation.

**9.** Procédé selon l'une des revendications précédentes, caractérisé en ce qu'on conduit l'étape a) en opérant en présence de quantités catalytiques d'un métal qui est plus électro-positif que le métal utilisé pour la réaction et que l'on utilise sous forme métallique et/ou sous forme ionique.

**10.** Procédé selon l'une des revendications précédentes, caractérisé en ce qu'on effectue l'étape a) en opérant en présence d'additifs activant les réactions de Grignard, notamment des agents ou produits contenant de l'halogène, comme l'iode, $CCl_4$.

**11.** Procédé selon l'une des revendications précédentes, caractérisé en ce qu'on conduit l'étape a) par la voie électrochimique.

**12.** Procédé selon l'une des revendications précédentes, caractérisé en ce que le ligand Y représente un atome d'halogène, en particulier le chlore, le brome ou l'iode.

**13.** Procédé selon l'une des revendications précédentes, caractérisé en ce qu'on effectue l'étape a) sous pression élevée dans un autoclave, jusqu'à une pression absolue d'au maximum 50 bars, avantageusement sous des pressions absolues d'l bar jusqu'à la pression d'équilibre déterminée par la température de réaction.

**14.** Composés de formule générale (I) :

$$CF_3 \cdot Zn \cdot X \cdot nL \quad (I)$$

dans laquelle X représente un halogène, avantageusement le chlore ou le brome ; n est un nombre valant 2 et L est un agent de complexation choisi parmi des amides d'acides carboxyliques éventuellement substitués.

**15.** Composés selon la revendication 14, caractérisés en ce que L est un amide d'acide carboxylique disubstitué sur l'azote, en particulier le N,N-diméthylformamide (DMF).

**16.** Composé selon la revendication 15, de formule :

$$CF_3 \cdot Zn \cdot Br \cdot 2 DMF$$

## Revendications pour l'Etat contractant suivant : ES

**1.** Procédé pour préparer $CF_3I$, caractérisé par :
   a) la réaction de $CF_3X$, sans addition de $SO_2$ ou de dithionite alcalin, avec un métal MT choisi dans le groupe constitué par le magnésium, le zinc, le cadmium, le bismuth, l'étain, dans un solvant,
   b) la réaction du mélange réactionnel résultant avec un composé I-Y pour obtenir $CF_3I$,
X représentant un atome d'halogène, en particulier le chlore ou le brome, et Y représentant l'iode ou un ligand plus électro-négatif que l'iode.

**2.** Procédé selon la revendication 1, caractérisé en ce qu'on utilise le zinc comme métal.

**3.** Modification du procédé selon la revendication 2, caractérisée par :
   a) la réaction de $CF_3X$ avec le zinc, sans addition de $SO_2$ ni d'un dithionite alcalin, dans un solvant et l'addition avant, pendant ou après la réaction d'un agent L de complexation au mélange réactionnel,
   a1) l'isolement du composé organo-métallique de formule générale (I) ainsi formé :

$$CF_3 \cdot Zn \cdot X \cdot nL \quad (I)$$

b) la réaction du composé de formule (I), isolé à l'étape a1), avec un composé I-Y pour obtenir $CF_3I$, X et Y ayant le sens précité, n étant un nombre valant 1 ou 2 et L représentant l'agent L de complexation.

4. Procédé selon l'une des revendications précédentes, caractérisé en ce qu'on effectue la réaction selon l'étape a) et/ou l'étape b) dans un solvant aprotique polaire ou dans un mélange de tels solvants.

5. Procédé selon la revendication 4, caractérisé en ce qu'on utilise un ou plusieurs solvants choisis dans l'ensemble constitué par les éthers cycliques ou acycliques, les amides d'acides carboxyliques éventuellement substitués, les lactames, nitriles, sulfoxydes, amines et/ou pyridines éventuellement substitué(e)s.

6. Procédé selon l'une des revendications 3 à 6, caractérisé en ce que l'agent L de complexation est un ligand choisi dans l'ensemble formé par les éthers cycliques ou acycliques, les amides d'acides carboxyliques éventuellement substitués, les lactames, nitriles, sulfoxydes, amines et/ou pyridines éventuellement substitués.

7. Procédé selon l'une des revendications 3 à 6, caractérisé en ce que le solvant et l'agent L de complexation sont identiques.

8. Procédé selon la revendication 7, caractérisé en ce qu'on utilise des amides d'acides carboxyliques disubstitués sur l'azote, en particulier le N,N-diméthylformamide (DMF) comme solvant et comme agent L de complexation.

9. Procédé selon l'une des revendications précédentes, caractérisé en ce qu'on conduit l'étape a) en opérant en présence de quantités catalytiques d'un métal qui est plus électro-positif que le métal utilisé pour la réaction et que l'on utilise sous forme métallique et/ou sous forme ionique.

10. Procédé selon l'une des revendications précédentes, caractérisé en ce qu'on effectue l'étape a) en opérant en présence d'additifs activant les réactions de Grignard, notamment des agents ou produits contenant de l'halogène, comme l'iode, $CCl_4$.

11. Procédé selon l'une des revendications précédentes, caractérisé en ce qu'on conduit l'étape a) en opérant de façon électrochimique.

12. Procédé selon l'une des revendications précédentes, caractérisé en ce que le ligand Y représente un atome d'halogène, en particulier le chlore, le brome ou l'iode.

13. Procédé selon l'une des revendications précédentes, caractérisé en ce qu'on effectue l'étape a) sous pression élevée dans un autoclave, jusqu'à une pression absolue d'au maximum 50 bars, avantageusement sous des pressions absolues d'l bar jusqu'à la pression d'équilibre déterminée par la température de réaction.

14. Procédé pour préparer des composés de formule générale (I) :

$$CF_3 \cdot Zn \cdot X \cdot nL \qquad (I)$$

dans laquelle X représente un halogène, avantageusement le chlore ou le brome, n est un nombre valant 2 et L est un agent de complexation choisi dans l'ensemble constitué par les amides d'acides carboxyliques éventuellement substitués, procédé caractérisé par :
a) la réaction de $CF_3X$ avec le zinc, sans addition de $SO_2$ ni d'un dithionite alcalin, dans un solvant, et l'addition au mélange réactionnel avant, pendant ou après la réaction, d'un agent L de complexation ;
a1) l'isolement du composé organo-métallique de formule générale (I) ainsi formé.

15. Procédé selon la revendication 14, caractérisé en ce que L est un amide d'acide carboxylique disubstitué sur l'azote, en particulier le N,N-diméthylformamide (DMF).

**16.** Procédé selon la revendication 15, caractérisé en ce que X représente le brome, L représente le diméthylformamide (DMF) et le composé ainsi préparé répond à la formule :

$CF_3 \bullet Zn \bullet Br \bullet 2\ DMF$